(19) Europäisches Patentamt
European Patent Office
Office européen des brevets

(11) Veröffentlichungsnummer: **0 230 018 B1**

(12) # EUROPÄISCHE PATENTSCHRIFT

(45) Veröffentlichungstag der Patentschrift: **26.08.92**

(51) Int. Cl.$^5$: **C07D 307/42**, C07D 307/46, C07D 307/54, C07D 307/45, C07D 409/12, A01N 43/08, A01N 43/10, //C07C249/00, C07C291/00,C07D261/04

(21) Anmeldenummer: **86117714.5**

(22) Anmeldetag: **19.12.86**

Die Akte enthält technische Angaben, die nach dem Eingang der Anmeldung eingereicht wurden und die nicht in dieser Patentschrift enthalten sind.

(54) **Pyrethroide, Verfahren zu ihrer Herstellung und ihre Verwendung zur Bekämpfung von Schädlingen sowie Vorprodukte für die Herstellung der Pyrethroide.**

(30) Priorität: **31.12.85 DE 3546371**

(43) Veröffentlichungstag der Anmeldung:
**29.07.87 Patentblatt 87/31**

(45) Bekanntmachung des Hinweises auf die Patenterteilung:
**26.08.92 Patentblatt 92/35**

(84) Benannte Vertragsstaaten:
**AT BE CH DE FR GB LI NL**

(56) Entgegenhaltungen:
**EP-A- 0 012 273**
**EP-A- 0 187 345**
**FR-A- 1 541 065**
**GB-A- 1 270 315**
**GB-B- 1 413 491**

(73) Patentinhaber: **BASF Aktiengesellschaft**
**Carl-Bosch-Strasse 38**
**W-6700 Ludwigshafen(DE)**

(72) Erfinder: **Wolf, Bernd, Dr.**
**Eichenstrasse 11**
**W-6704 Mutterstadt(DE)**
Erfinder: **Theobald, Hans, Dr.**
**Oueichstrasse 6**
**W-6703 Limburgerhof(DE)**
Erfinder: **Becker, Rainer, Dr.**
**Im Haseneck 22**
**W-6702 Bad Duerkheim(DE)**
Erfinder: **Goetz, Norbert, Dr.**
**Schoefferstrasse 25**
**W-6520 Worms 1(DE)**
Erfinder: **Himmele, Walter, Dr.**
**Eichenweg 14**
**W-6909 Walldorf(DE)**
Erfinder: **Kropp, Rudolf**
**Sprottauer Strasse 2**
**W-6703 Limburgerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**PESTICIDE SCIENCE, Band 2, Nr. 6, November-Dezember 1971, Seiten 243-248; M. ELLIOTT et al.: "The Pyrethrins and Related Compounds. XIII Insecticidal methyl-, alkenyl and benzyl-substituted furfuryl and furyl-methyl chrysanthemates"**

## Beschreibung

Die Erfindung betrifft neue Pyrethroide, nämlich Ester von Benzylfurylcarbinolen, Verfahren und Vorprodukte zu ihrer Herstellung, Schädlingsbekämpfungsmittel, die diese Ester als Wirkstoffe enthalten, sowie Verfahren zur Bekämpfung von Schädlingen mit diesen Wirkstoffen.

Es ist bekannt, daß gewisse Wirkstoffe, nämlich Ester von Benzylfurylcarbinolen, sogenannte Pyrethroide, zur Bekämpfung von Insekten geeignet sind, vgl. südafrikanische Patentanmeldung 67/5027; Pestic. Sci. 2 (6), 243, Pestic. Sci. 5 (4), 491; japanische Patentveröffentlichungen 71/35872 und 80/17323.

Pyrethroide, die sich Von der 3,3-Dimethylcyclopropan-1-carbonsäure mit einer 2',2'-disubstituierten Vinylgruppe in 2-Position ableiten, sind in der EP-A 12 273, GB-B-1 270 315 und GB-B-1 413 491 offenbart.

Aus der älteren Anmeldung EP-A 187 345 ist bekannt, in 5-Stellung zweifach durch Hydroxymethyl bzw. Acetyloxymethyl substituierte Isoxazoline, ausgehend von speziell substituierten Phenylacetaldehyden herzustellen.

Es bestand nun die Aufgabe, Pyrethroide mit verbesserter insektizider Wirkung herzustellen.

Demgemäß wurden neue Ester und die zu ihrer Herstellung erforderlichen Vorprodukte sowie Verfahren zur Gewinnung der Vorprodukte gefunden, die gegenüber den bekannten Wirkstoffen bzw. den zu ihnen führenden Syntheseschritten bemerkenswerte Vorteile aufweisen.

Die Ester und ihre Vorprodukte (R = CHO) können durch die nachstehende Formel I gemeinsam dargestellt werden:

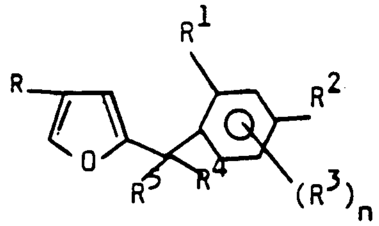

(I),

in der

| | |
|---|---|
| $R^1$, $R^2$ und $R^3$ | gleiche oder verschiedene Substituenten der Bedeutung Wasserstoff, Halogen, Alkyl, Halogenalkyl, Alkoxy, Halogenalkoxy, Halogenalkylthio, Alkenyl oder Halogenalkenyl mit jeweils bis zu 6 C-Atomen sind, wobei n die Zahl 1, 2 oder 3 ist, falls $R^3$ vom Wasserstoff verschieden ist, |
| $R^4$ und $R^5$ | Wasserstoff oder Alkyl mit bis zu 6 C-Atomen, |
| R | die Bedeutung -CHO oder $CHR^6OA$ hat, wobei |
| $R^6$ | für Wasserstoff, Cyano, Alkyl, Alkenyl, Halogenalkenyl oder Alkinyl mit jeweils bis zu 6 Kohlenstoffatomen oder die Carbonamidgruppe steht und |
| A | die Acylgruppe einer 3,3-Dimethylcyclopran-1-carbonsäure bedeutet, die in 2-Stellung durch eine 2',2'-disubstituierte Vinylgruppe substituiert ist, |

mit der Maßgabe, daß, wenn A den Rest der 3,3-Dimethylcyclopropan-1-carbonsäure bedeutet, die in 2-Stellung eine 2',2'-Dimethylvinylgruppe trägt, $R^2$ nicht Methyl, Chlor, Brom oder Methoxy und $R^1$ und $R^3$ nicht Wasserstoff sind und ferner $R^1$ nicht Wasserstoff oder Methyl bedeutet, wenn $R^2$ und $R^3$ gleichzeitig für Wasserstoff stehen.

Die Ester der Formel 1 können durch Umsetzung von Carbonsäuren AOH mit den Benzylfurylcarbinolen der Formel I in bekannter Weise gewonnen werden. Anstelle der Carbonsäuren können die entsprechenden Säurehalogenide oder evtl. -anhydride verwendet werden.

Für die Erfindung kommt es somit wesentlich auf die Natur der Vorprodukte bzw. deren Zugänglichkeit an. Hierzu ist im einzelnen das Folgende zu sagen:

Es ist bekannt, daß Benzylfurylcarbinole wie z. B. 2-Benzyl-4-hydroxymethylfuran einen Bestandteil Künstlicher Pyrethrum-Wirkstoffe bilden [Wegler, Chemie der Pflanzenschutz- und Schädlingsbekämpfungsmittel, Vol. 7 (1981)]. Erstaunlicherweise ist jedoch - im Gegensatz zur Säurekomponente - die Variation der Benzylfurylcarbinole durch Substitution des Benzylrestes auf einige wenige Beispiele beschränkt geblieben. (Pestic. Sci. 2 (6), 243; JP-AS 71/35872; JP-AS 71/04198; südafrikanische Patentanmeldung 67/5027)

Die Herstellung der erfindungsgemäßen 2-Benzylfurylverbindungen I erfolgt über Furylcarbinole der allgemeinen Formel Ia

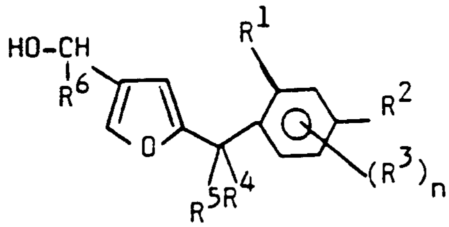

(Ia),

in der n und die Reste $R^1$ bis $R^6$ die obengenannte Bedeutung haben.

Zur Herstellung der Carbinole ist im einzelnen das Folgende zu sagen:

Es ist zwar bekannt, daß Furane aus 1,4-Diketonen durch Cyclisierung hergestellt werden können [Comprehensive Heterocyclic Chemistry Vol. 4, S. 657 ff (1984)). Insbesondere 2,4-disubstituierte Furane sind jedoch mangels entsprechender Ausgangsprodukte schwer zugänglich. Aus diesem Grunde werden schon für die Herstellung der einfachsten Verbindung, nämlich des 2-Benzyl-4-hydroxymethyl-furans, verschiedene Verfahren empfohlen (Elliott, Janes, Pearson; J. Chem. Soc. C, 1971, 2551; DE-OS 21 22 823; Wegler, Chemie der Pflanzenschutz- und Schädlingsbekämpfungsmittel, Vol. 7, S. 76 und dort zit. Lit.). Alle diese Verfahren zeigen jedoch schwerwiegende Nachteile (vielstufige Synthesen mit mäßiger Gesamtausbeute, Sicherheitsprobleme durch hohe Temperaturen, Isomerenbildung).

Die Furylcarbinole der allgemeinen Formel Ia, bei denen $R^6$ für Wasserstoff steht, erhält man dadurch, daß man ein entsprechendes Isoxozolin der allgemeinen Formel II hydriert und gegebenenfalls mit Säure behandelt

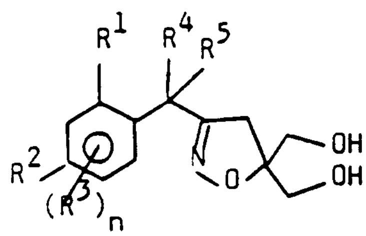

(II).

Abhängig von der speziellen Verfahrensweise kann dies direkt geschehen oder es kann als Zwischenstufe das Hydroxyketon III isoliert und in einem anschließenden Schritt das gewünschte Furylcarbinol erhalten werden

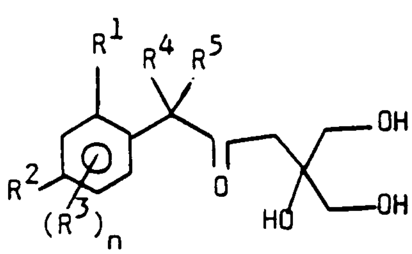

(III).

Die katalytische Hydrierung wird unter den üblichen Bedingungen durchgeführt. Als Katalysator kommen alle Metalle der 8. Nebengruppe in fein verteilter Form oder auf einem Träger oder als Verbindung in Betracht. Auch Gemische können verwendet werden. Besonders bewährt hat sich Platin(IV)-oxid.

Als Lösungsmittel kommen in Frage: Ether (wie z.B. Tetrahydrofuran oder Dioxan), Alkohole (wie z. B. Methanol oder Ethanol), Nitrile (wie z.B. Acetonitril oder Propionitril) oder Ketone (wie z.B. Aceton oder Methylethylketon). Die Hydrierung kann vorteilhaft in Gegenwart von Säuren (wie z.B. Essigsäure oder Borsäure) durchgeführt werden.

Furylcarbinole der allgemeinen Formel Ia, in der $R^6$ Cyano, Alkyl, Alkenyl, Halogenalkenyl oder Alkinyl mit bis zu 6 C-Atomen oder die Carbonamidgruppe bedeutet erhält man dadurch, daß man zunächst die in $R^6$ unsubstituierten Furylcarbinole Ib zu den entsprechenden neuen Furanaldehyden Ic oxidiert.

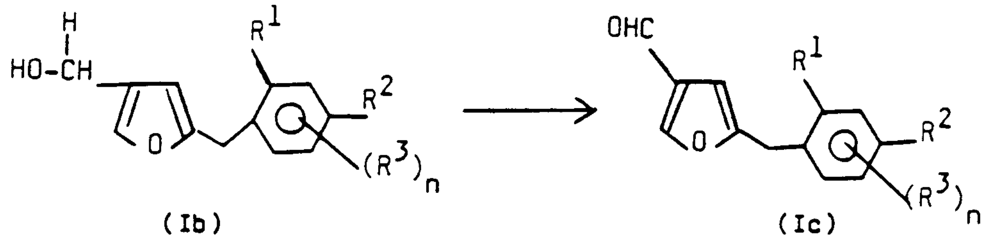

(Ib) (Ic)

Als Oxidationsmittel in Frage kommen alle gängigen Oxidationsmittel, die primäre Alkohole in Aldehyde überführen, jedoch nicht so stark sauer sind, daß eine Spaltung des Furanringes eintritt (Houben-Weyl, Methoden der Organischen Chemie, Band E3, S. 265 ff). Besonders geeignet sind Verbindungen mit Übergangsmetallen in höherer Oxidationsstufe, z.B. Pyridindichromat.

Die Furanaldehyde werden in einem anschließenden Reaktionsschritt wiederum zu den - nunmehr in $R^6$ substituierten - Furylcarbinolen umgesetzt. Dabei wird

a) wenn $R^6$ Cyano bedeuten soll, der Furanaldehyd in üblicher Weise mit Blausäure (oder einem Metallcyanid in Gegenwart einer Säure) zur Reaktion gebracht, d.h. der Cyanhydrinreaktion unterworfen,

b) wenn $R^6$ für Alkyl, Alkenyl, Halogenalkenyl oder Alkinyl steht, der Furanaldehyd mit einem entsprechenden Metallorganyl $R^6$-M umgesetzt.

Als Metallcyanide werden bevorzugt Alkalimetallcyanide wie z.B. Natrium- oder Kaliumcyanid verwendet, wobei gegebenenfalls ein Hilfsreagenz z. B. $NaHSO_3$ oder ein Phasentransferkatalysator zu dem Zweiphasensystem gefügt wird.

Als Metallorganyle eignen sich die entsprechenden metallorganischen Verbindungen mit Metallen der 1. Hauptgruppe sowie die entsprechenden Grignardverbindungen, wobei letztere, z.B. Vinylmagnesiumchlorid, aufgrund ihrer leichteren Handhabung bevorzugt werden.

Zur Erfindung gehört weiterhin ein Verfahren zur Herstellung der als Zwischenprodukte erforderlichen Isoxazoline der allgemeinen Formel IV

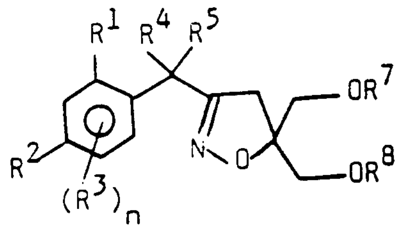

(IV),

wobei $R^1$ bis $R^5$ entsprechende Bedeutung haben und $R^7$ und $R^8$ gleich oder verschieden sind und z.B. für Wasserstoff, Alkyl, Alkylcarbonyl, Arylcarbonyl oder eine andere abspaltbare Hydroxy-Schutzgruppe stehen, mit der Maßgabe, daß $R^7$ und $R^8$ nicht für Acetyl stehen, wenn

$R^1$, $R^2$ und $R^3$ Wasserstoff;

$R^1$ und $R^2$ Halogen und $R^3$ Wasserstoff;

$R^1$ und $R^2$ Wasserstoff, n = 1 und $R^3$ Trifluormethyl bedeuten;

sowie ferner mit der Maßgabe, daß $R^7$ und $R^8$ nicht für Wasserstoff stehen, wenn $R^1$, $R^2$ und $R^3$ Wasserstoff oder $R^1$ und $R^3$ Wasserstoff und $R^2$ Halogen bedeuten.

Die Funktion von $R^7$ und $R^8$ hat ersichtlich nur Hilfscharakter.

Zur Herstellung der Isoxazoline geht man zweckmäßig von entsprechenden Phenylacetaldehyden V aus. Diese lassen sich mit Hydroxylamin oder einem Hyroxylammoniumsalz in Gegenwart eines Hilfsmittels, z.B. einer Base, in Oxime IV überführen

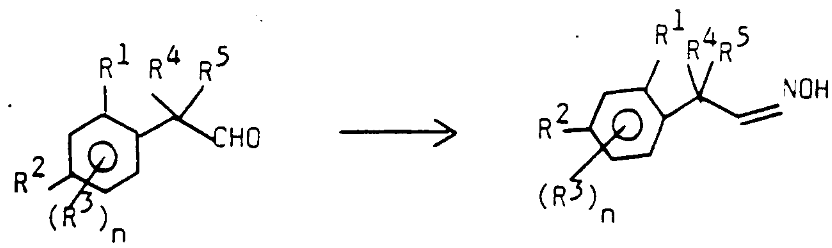

(V) (VI).

Nach allgemein bekannten Methoden ("The Nitrile Oxides" von Grundmann, Grünanger, 1. Aufl. 1971, S. 96 ff) können daraus Nitriloxide (VII) erhalten und mit Olefinen VIII zu den gewünschten Isoxazolinen umgesetzt werden [vgl. auch Heterocycles, Bd. 12, S. 1243 ff (1979); Houben-Weyl, Methoden der Organischen Chemie, 14. Aufl., Band 10/3, S. 837, Stuttgart 1965]:

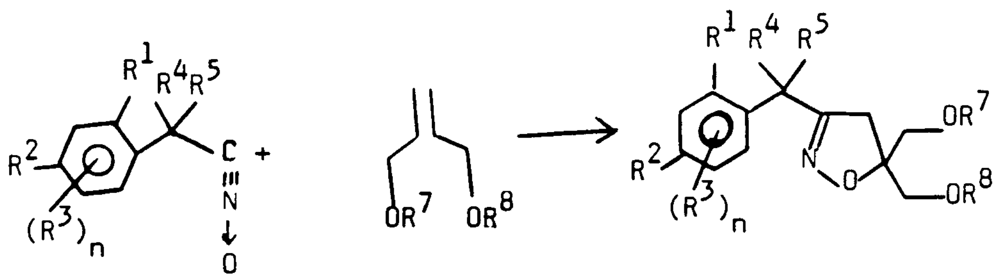

(VII) (VIII) (IV).

Die Isolierung der Nitriloxide ist nicht unbedingt erforderlich. Sie werden vorteilhaft in situ mit den Alkenylverbindungen umgesetzt.

Die zur Darstellung der Isoxazoline verwendeten Nitriloxide und die Alkenylverbindungen können in stöchiometrischer Menge oder jeweils einer der Reaktionspartner im Überschuß eingesetzt werden.

Als Lösungsmittel für die Umsetzung eignen sich z.B. die jeweilige Alkenylverbindung selbst, ferner aromatische Verbindungen (z.B. Benzol, Toluol, Xylol), halogenierte aromatische Verbindungen, Ketone (z.B. Aceton, Methylethylketon), Ether (z.B. Diethylether, Tetrahydrofuran, Dioxan) und chlorierte aliphatische Kohlenwasserstoffe (z.B. Methylenchlorid, Chloroform).

1. Herstellungsbeispiel für ein Oxim

Zu 160 g p-Fluorphenylacetaldehyd in 500 ml Methylenchlorid werden 113 g Hydroxylammoniumchlorid in 300 ml Wasser gegeben. Unter Rühren tropft man eine Lösung von 87 g Natriumcarbonat in 300 ml Wasser bei Raumtemperatur langsam zu und läßt 2 Tage nachreagieren. Die organische Phase wird abgetrennt, mit Wasser gewaschen, über $Na_2SO_4$ getrocknet und nach Filtration vom Lösungsmittel befreit. Man erhält 161 g p-Fluorphenylacetaldoxim vom Schmelzpunkt 113 - 115 °C
200-MHz-NMR-Spektrum in $CDCl_3$:

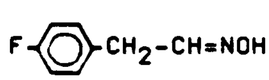

(Isomerengemisch cis/trans = 1 : 1)

δ[ppm] = 3,51 und 3,72 (2 Dubletts, 2H, J = 7 Hz, $CH_2$)
6,9 und 7,54 (2 Tripletts, 1H, J = 7 Hz, =CH)
6,95 - 7,32 (4H, aromat. Protonen)
8,52 und 9,02 (1H, OH)

2. Herstellungsbeispiel für ein Isoxazolin

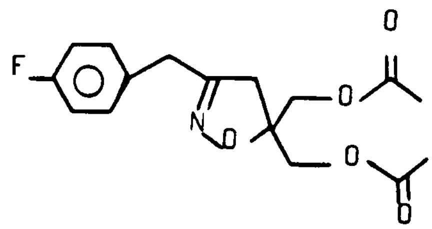

203,5 g p-Fluorphenylacetaldoxim und 255 g 2-Methylen-propan-1,3-diol-diacetat werden in 1 000 ml Diethylether gelöst. Unter starkem Rühren tropft man bei 20 - 30 ° C (Kühlung) 1 000 ml einer 10prozentigen Natriumhypochlorit-Lösung langsam hinzu. Nach 20stündigem Rühren wird die organische Phase abgetrennt, mit Wasser gewaschen, über Natriumsulfat getrocknet und das Lösungsmittel abgezogen. Überschüssiges Isobutylendiacetat destilliert man über eine Brücke ab (Kp. 70 ° C/1 Torr). Ausbeute: 312 g (zähes Öl) 3-(p-Fluorbenzyl)-5,5-bis(acetoxymethyl)-isoxazolin.

300-MHz-NMR-Spektrum in $CDCl_3$:

δ[ppm] = 2,03 (6H, 2 × $OCH_3$)
2,75 (2H, $CH_2$)
3,65 (2H, $CH_2$)
4,15 (4H, 2 × $OCH_2$)
6,95 - 7,1 (2H, aromat. Protonen)
7,15 - 7,3 (2H, aromat. Protonen)

3. Beispiel für die Abspaltung der Schutzgruppen an dem Isoxazolin

138 g 3-(p-Fluorbenzyl)-5,5-bis(acetoxymethyl)isoxazolin werden mit einer Lösung von 58,3 g KOH in 900 ml Ethanol versetzt und 12 Stunden unter Rückfluß erhitzt. Nach dem Abkühlen filtriert man das ausgefallene Salz ab und entfernt das Lösungsmittel bei vermindertem Druck. Nach Zugabe von Aceton wird erneut ausgefallener Feststoff abgesaugt und das Filtrat eingeengt. Das erhaltene Öl kann säulenchromatographisch [Kieselgel; Elutionsmittel: Toluol/Aceton (anfangs 9/1, später 1/1)] gereinigt werden. Man erhält 83 g 3-(p-Fluorbenzyl)-5,5-bis(hydroxymethyl)isoxazolin vom Schmelzpunkt 93 - 97 ° C.

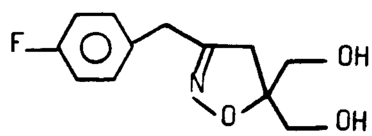

| Analyse: | | | | |
|---|---|---|---|---|
| Ber. | C 60,20 | H 5,9 | N 5,85 | F 7,94 |
| Gef. | C 60,3 | H 5,6 | N 5,9 | F 8,0 |

270-MHz-NMR-Spektrum in $CDCl_3$:

δ[ppm] = 2,3 (2H, OH, breit)
2,8 (2H)
3,5 - 3,75 (2H, 2H, 2H)
6,95 - 7,25 (4H)

4. Herstellungsbeispiel für ein Furylcarbinol ($R^6$ = H)

67,9 g 3-(p-Fluorbenzyl)-5,5-bis(hydroxymethyl)isoxazolin werden in 400 ml Tetrahydrofuran, 80 ml Eisessig und 40 ml Wasser aufgenommen und mit 1,5 g Platin(IV)-oxidhydrat versetzt. Bei 25 - 30 ° C wird 5 - 6 Stunden lang hydriert, wobei eine leicht exotherme Reaktion stattfindet (Kühlung). Nach beendeter Wasserstoffaufnahme filtriert man den Katalysator ab, engt ein und rührt den Rückstand in 400 ml

Methylenchlorid mit 400 ml 10prozentiger Salzsäure 1 Stunde lang bei Raumtemperatur. Die organische Phase wird abgetrennt, neutral gewaschen, über Natriumsulfat getrocknet und eingeengt. Nach säulenchromatographischer Reinigung über Kieselgel mit Toluol/Aceton (9/1) als Elutionsmittel erhält man 37 g 3-Hydroxymethyl-5-(p-Fluorbenzyl)furan (Schmelzpunkt: 45 - 47° C).

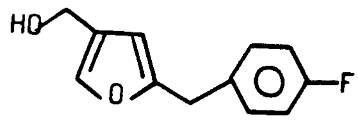

| Analyse: | | | |
|---|---|---|---|
| Ber.<br>Gef. | C 69,89<br>C 69,2 | H 5,38<br>H 5,6 | F 9,21<br>F 9,2 |

250-MHz-NMR-Spektrum in $CDCl_3$:
δ [ppm] = 2,1 (1H, breit); 3,87 (2H); 4,42 (2H); 6,01 (1H); 6,9 - 7,0 (2H); 7,1 - 7,2 (2H); 7,26 (1H)

5. Herstellungsbeispiel für einen Furanaldehyd

Zu 1 100 ml Pyridin werden bei 0° C portionsweise 59 g Chrom(VI)-oxid gegeben, wobei sich ein voluminöser gelber Niederschlag bildet. Nach 15 Minuten tropft man - ebenfalls bei 0° C - eine Lösung von 37 g 3-Hydroxymethyl-5-(p-fluorbenzyl)furan in 100 ml Pyridin ein, rührt 2 Stunden bei Raumtemperatur und läßt einige Zeit bei dieser Temperatur stehen. Das dunkel gefärbte Reaktionsgemisch wird zu 3 000 ml Wasser gegeben und die Lösung mehrmals mit Ether extrahiert. Die vereinigten Etherlösungen werden mit verdünnter Salzsäure und anschließend mit Wasser gewaschen, über Natriumsulfat getrocknet und eingeengt. Nach säulenchromatographischer Reinigung über Kieselgel mit Toluol als Elutionsmittel erhält man 23 g 3-Formyl-5-(p-fluorbenzyl)furan ($n_D^{22}$ : 1,5415).

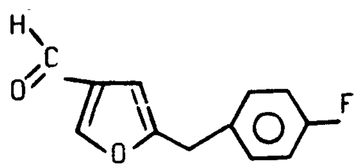

250-MHz-NMR-Spektrum in $CDCl_3$:
δ [ppm] = 3,95 (2H); 6,39 (1H); 6,94 - 7,05 (2H); 7,13 - 7,23 (2H); 7,95 (1H); 9,86 (1H).

6. Herstellungsbeispiel für ein Furylcarbinol ($R^6 \neq H$)

46,5 ml einer 1,5 molaren Lösung von Vinylmagnesiumchlorid in Tetrahydrofuran werden in 60 ml absolutem Tetrahydrofuran bei 0° C vorgelegt. Unter Rühren und Feuchtigkeitsausschluß tropft man 11,9 g 3-Formyl-5-(p-Fluorbenzyl)furan in 60 ml absolutem Tetrahydrofuran bei 0 - 10° C zu und läßt 15 Stunden bei Raumtemperatur rühren. Das Reaktionsgemisch wird vorsichtig mit 100 ml einer gesättigten Ammoniumchlorid-Lösung und 20 ml Eisessig versetzt, wobei sich der Niederschlag auflöst. Nach dreimaligem Ausschütteln mit Ether werden die vereinigten Etherextrakte mit Wasser gewaschen, über Natriumsulfat getrocknet und unter vermindertem Druck eingeengt. Das erhaltene Öl (14 g) wird säulenchromatographisch über Kieselgel mit Toluol/Aceton (9/1) als Elutionsmittel gereinigt. Es ergeben sich 7,1 g (Öl, $n_D^{22}$ : 1,5370) 3-(1-Hydroxy-2-propenyl)-5-(p-fluorbenzyl)furan.

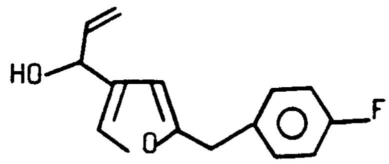

200-MHz-NMR-Spektrum in $CDCl_3$:
δ [ppm] = 1,87 (1H, breit); 3,93 (2H); 5,07 - 5,43 (1H, 1H, 1H); 5,97 - 6,17 (1H, 1H); 6,94 - 7,08 (2H); 7,16 - 7,33 (2H, 1H).

Tabelle 1 - Oxime

| $R^1$ | $R^2$ | $R^3$ | $R^4$ | $R^5$ | Schmelzpunkt [°C] | Meßfrequenz [MHz] | $^1$H-NMR-Daten ($CDCl_3$) $\delta$ [ppm] |
|---|---|---|---|---|---|---|---|
| H | H | H | $CH_3$ | H | Öl | 270 | cis/trans-Isomerengemisch: 1.45 (2H), 3.68 u. 4.46 (1H), 6.82 u. 7.52 (1H), 7.18 - 7.38 (5H) |
| F | H | H | H | H | | 300 | cis/trans-Isomerengemisch: 3.57 u. 3.76 (2H), 6.86 u. 7.53 (1H), 6.9 - 7.3 (4H) |
| H | H | 3-F | H | H | | 300 | cis/trans-Isomerengemisch: 3.5 u. 3.74 (2H), 6.7 - 7.38 (4H), 7.5 u. 7.64 (1H), 8.3 u. 8.83 (1H, breit) |
| Cl | H | 6-F | H | H | | 300 | cis/trans-Isomerengemisch: 3.72 u. 3.91 (2H), 6.72 u. 7.52 (1H), 7.01 (1H), 7.2 (2H) |
| Cl | H | H | H | H | | 300 | cis/trans-Isomerengemisch: 3.66 u. 3.85 (2H), 6.86 u. 7.57 (1H), 7.22 (3H), 7.37 (1H) |
| H | H | 3-Cl | H | H | 65 - 73 | 300 | cis/trans-Isomerengemisch: 3.52 u. 3.73 (2H), 6.88 u. 7.52 (1H), 7.1 (1H), 7.23 (3H), 9.22 u. 9.7 (1H, breit) |
| Cl | Cl | H | H | H | 103 - 110 | 270 | cis/trans-Isomerengemisch: 3.63 u. 3.8 (2H), 6.85 u. 7.55 (1H), 7.13 - 7.28 (2H), 7.4 (1H), 8.35 u. 8.92 (1H, breit) |
| H | $OCH_3$ | 3-$OCH_3$ | H | H | 80 - 87 | 200 | cis/trans-Isomerengemisch: 3.53 u. 3.75 (2H), 3.93 (6H), 6.75 - 6.9 (3H), 6.95 u. 7.6 (1H) |
| H | H | 3-$CF_3$ | H | H | | 300 | cis/trans-Isomerengemisch: 3.58 u. 3.79 (2H), 6.87 u. 7.67 (1H), 7.3 - 7.62 (4H) |

Tabelle 2 - Isoxazoline

| $R^1$ | $R^2$ | $R^3$ | $R^4$ | $R^5$ | $R^7$ | $R^8$ | Meßfrequenz [MHz] | $^1$H-NMR-Daten ($CDCl_3$) $\delta$ [ppm] |
|---|---|---|---|---|---|---|---|---|
| H | H | H | $CH_3$ | H | Ac | Ac* | 300 | 1.53 (3H, Dublett), 2.0 (3H), 2.03 (3H), 2.71 (2H, Multiplett), 3.82 (1H, Quartett), 4.12 (2H), 4.15 (2H), 7.18 - 7.42 (5H) |
| H | H | H | $CH_3$ | $CH_3$ | Ac | Ac | 300 | 1.6 (6H), 2.06 (6H), 2.65 (2H), 4.12 (4H), 7.17 - 7.44 (5H) |
| H | H | 3-F | H | H | Ac | Ac | 300 | 2.03 (6H), 2.77 (2H), 3.68 (2H), 4.06 - 4.23 (4H), 6.8 - 7.4 (4H) |
| Cl | Cl | H | H | H | Ac | Ac | 200 | 2.06 (6H), 2.8 (2H), 3.81 (2H), 4.16 (4H), 7.25 (2H), 7.43 (1H) |
| H | $OCH_3$ | 3-$OCH_3$ | H | H | Ac | Ac | 300 | 2.05 (6H), 2.77 (2H), 3.62 (2H), 3.89 (6H), 4.16 (4H), 6.7 - 6.9 (3H) |
| H | H | 3-$CF_3$ | H | H | Ac | Ac | 300 | 2.01 (6H), 2.8 (2H), 3.75 (2H), 4.04 - 4.23 (2H) (2H, 2H), 7.4 - 7.68 (4H) |
| H | Cl | H | H | H | Ac | Ac | 300 | 2.04 (6H), 2.75 (2H), 2.65 (2H), 4.14 (4H), 7.02 - 7.42 (4H) |

*Ac = Acetyl

Tabelle 2 - <u>Isoxazoline</u>

| $R^1$ | $R^2$ | $R^3$ | $R^4$ | $R^5$ | Schmelzpunkte [°C] | Meßfrequenz [MHz] | $^1$H-NMR-Daten[$CDCl_3$] δ [ppm] | Analyse |
|---|---|---|---|---|---|---|---|---|
| H | H | H | $CH_3$ | H | 84 - 85 | 200 | 1.52 (3H, Dublett, J = 8Hz), 2.6(2H, breit), 2.7 (2H), 3.45 - 3.71 (4H), 3.78 (1H, Quart., J = 8Hz), 7,18 -7,42 (5H) | Ber. C 66.36 H 7.28 O 20.4 N 5.95<br>Gef. C 66.4 H 7.3 O 20.6 N 5.9 |
| H | H | H | $CH_3$ | $CH_3$ | | 300 | 1.57 (6H), 2.65 (2H), 3.26 (2H, breit) 3.47 - 3.68 (2H, 2H), 7.14 - 7.43 (5H) | Ber. C 67.5 H 7.7 N 5.6<br>Gef. C 67.3 H 7.6 N 5.5 |
| F | H | H | H | H | | 300 | *2.72 (2H), 3.35 (4H), 3.65 (2H), 4.99 (2H,breit), 7.1 - 7.4 (4H) | |
| H | H | 3-F | H | H | | 300 | 2.78 (2H), 3.1 (2H,breit) 3.48 - 3.71 (6H), 6.86 - 7.03 (3H), 7.2 - 7.35 (1H) | |
| Cl | H | 6-F | H | H | | 200 | 2.72 (2H,breit), 2.9 (2H), 3.53 - 3.73 (2H, 2H), 3.87 (2H), 6.97 - 7.11 (1H), 7.19 - 7.3(2H) | |

Tabelle 2 - <u>Isoxazoline</u>

| $R^1$ | $R^2$ | $R^3$ | $R^4$ | $R^5$ | Schmelz-punkte [°C] | Meß-frequenz [MHz] | $^1H$-NMR-Daten[$CDCl_3$] δ [ppm] | Analyse |
|---|---|---|---|---|---|---|---|---|
| Cl | H | H | H | H | | 200 | 2.7 (2H, breit), 2.85 (2H), 3.52 - 3.77 (2H,2H), 3.83 (2H), 7.2 - 7.48(4H) | |
| H | H | 3-Cl | H | H | Öl | 270 | 2.8 (2H), 2.9 (2H,breit) 3.5 - 3.71 (2H,2H,2H), 7.05 - 7.35 (1H, 3H) | Ber. C 56.37 H 5.52 O 18.77 N 5,48 Cl 13.86<br>Gef. C 57.5 H 5.8 O 18.2 N 5.4 Cl 13.4 |
| Cl | Cl | H | H | H | 102 - 111 | 300 | 2.1 (2H, breit), 2.86 (2H) 3.58 (2H), 3.71 (2H),3.78 (2H), 7.23 (2H), 7.41 (1H) | |
| H | $OCH_3$ | 3-$OCH_3$ | H | H | 105 - 108 | 270 | 2.75 (2H), 3.6 (4H, 2H), 3.82 (6H), 6.65-6.84(3H) | Ber. C 59.78 H 6.81 O 28.44 N 4.98<br>Gef. C 60.2 H 6.8 O 28.7 N 4.6 |
| H | H | 3-$CF_3$ | H | H | | 270 | 2.42 (1H, breit), 2.81(2H), 3.51 - 3.75 (2H, 2H, 2H), 7.37 - 7.59 (4H) | |
| H | Cl | H | H | H | | 300 | 2.5 (2H, breit), 2.79 (2H), 3.5 - 3.7 (2H, 2H, 2H), 7.13 - 7.22 (2H), 7.25 - 7.34 (2H) | |

$R^7$, $R^8$ = H

* in DMSO-$d_6$ aufgenommen

Tabelle 3 - Furylcarbinole

| $R^1$ | $R^2$ | $R^3$ | $R^4$ | $R^5$ | $R^6$ | Brechungs-index | Meßfrequenz [MHz] | $^1$H-NMR-Daten [$CDCl_3$] $\delta$ [ppm] | Analyse |
|---|---|---|---|---|---|---|---|---|---|
| H | H | H | $CH_3$ | H | H | $n_D^{24}$: 1.5467 | 200 | 1.56 (3H, Dublett, J = 7Hz), 2.05 (1H, breit), 4.06 (1H, Quartett, J = 7Hz), 4.43(2H), 6.1 (1H), 7.15 - 7.38 (5H,1H), | Ber. C 77.2 H 6.98 O 15.82<br>Gef. C 76.5 H 7.20 O 16.6 |
| H | H | H | $CH_3$ | $CH_3$ | H | | 360 | 1.39 (6H), 2.72 (1H, breit), 4.23 (2H), 5.91 (1H), 6.88 - 7.15 (5H, 1H) | |
| F | H | H | H | H | H | | 300 | 2.32 (1H, breit), 3.95 (2H), 4.42 (2H), 6.04 (1H), 6.97 -7.27 (4H, 1H) | |
| H | H | 3-F | H | H | H | | 300 | 3.25 (1H, breit), 3.92 (2H), 4.45 (2H), 6.06 (1H), 6.84 - 7.05 (3H), 7.19 - 7.34 (1H,1H) | |
| Cl | H | 6-F | H | H | H | | 300 | 1.92 (1H, breit), 4.11 (2H), 4.43 (2H), 6.02 (1H), 6.95 - 7.05 (1H), 7.12 - 7.23 (2H), 7.27 (1H) | |

Tabelle 3 - <u>Furylcarbinole</u>

| $R^1$ | $R^2$ | $R^3$ | $R^4$ | $R^5$ | $R^6$ | Brechungs-index | Meßfre-quenz [MHz] | $^1H$-NMR-Daten [$CDCl_3$] $\delta$ [ppm] | Analyse |
|---|---|---|---|---|---|---|---|---|---|
| Cl | H | H | H | H | H | | 300 | 2.05 (1H, breit), 4.04 (2H), 4.43 (2H), 6.04 (1H), 7.17 (3H), 7.27 (1H), 7.3 - 7.38 (1H) | |
| H | H | 3-Cl | H | H | H | $n_D^{22}$ : 1.5631 | 200 | 1.73 (1H, breit), 3.9 (2H), 4.47 (2H), 6.06 (1H), 7.05 - 7.25 (4H), 7.3 (1H) | Ber. C 64.73 H 4.98 O 14,37 Cl 15,9<br>Gef. C 65.3 H 5.0 O 15.0 Cl 15.1 |
| Cl | Cl | H | H | H | H | | 200 | 2.26 (1H, breit), 4.03 (2H), 4.47 (2H), 6.07 (1H), 7.15 (2H), 7.31 (1H), 7.4 (1H) | Ber. C 56.06 H 3.92 O 12.45 Cl 27.58<br>Gef. C 56.0 H 4.0 O 12.6 Cl 27.5 |
| H | $OCH_3$ | 3-$OCH_3$ | H | H | H | 62 - 64 | 200 | 2.1 (1H, breit), 3.83 (6H), 3.87 (2H), 4.47 (2H), 6.03 (1H), 6.79 (3H), 7.28 (1H) | |
| H | H | 3-$CF_3$ | H | H | H | | 300 | 3.98 (2H), 4.48 (2H), 6.07 (1H), 7.3 (1H), 7.33 - 7.6 (4H) | |
| H | F | H | H | H | -C≡CH | | 200 | 2.26 (1H), 2.57 (1H), 3.93 (2H), 5.33 (1H), 6.13 (1H), 6.93 - 7.07 (2H), 7.14 - 7.27 (2H), 7.44 (1H) | |

Unter entsprechender Abwandlung der Herstellvorschriften können zum Beispiel folgende Furylcarbinole erhalten werden:

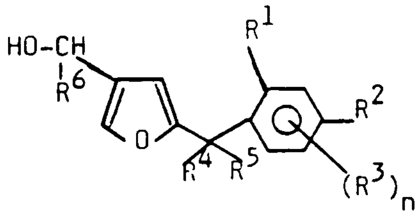

Tabelle 4

| $R^1$ | $R^2$ | $R^3$ | n | $R^4$ | $R^5$ | $R^6$ |
|---|---|---|---|---|---|---|
| H | F | H | - | H | H | $CH_3$ |
| H | F | H | - | $CH_3$ | H | H |
| H | F | H | - | H | H | $CH(CH_3)_2$ |
| H | F | H | - | $C_2H_5$ | $C_2H_5$ | H |
| H | F | H | - | H | H | CN |
| H | F | H | - | H | H | $n-C_4H_9$ |
| H | F | H | - | H | H | $CF=CF_2$ |
| H | H | 3-F | 1 | H | H | CN |
| H | H | 3-F | 1 | H | H | C≡CH |
| H | F | 3-Cl | 1 | H | H | H |
| H | tert.-$C_4H_9$ | H | - | H | H | $C_2H_5$ |
| H | $CF_3$ | H | - | H | H | H |
| $CF_3$ | H | H | - | H | H | H |
| H | $CF_3$ | H | - | H | H | CN |
| H | $CF_3$ | H | - | H | H | C=CH |
| H | F | 3-$CF_3$ | 1 | H | H | H |
| F | F | H | - | H | H | H |
| H | Cl | 3-Cl | 1 | H | H | CN |
| Cl | Cl | H | - | H | H | C≡CH |
| H | H | Cl(3,5) | 2 | H | H | $C=CH_2$ |
| F | F | F | 3 | H | H | H |
| F | F | F | 3 | H | H | C≡CH |
| H | F | 3F | 1 | H | H | H |
| H | H | 3F | 1 | H | H | CN |
| H | $n-C_4H_9$ | H | - | $CH(CH_3)_2$ | H | H |
| $C_2H_5$ | $C_2H_5$ | H | - | $CH_3$ | $CH_3$ | $CH_3$ |
| H | $OCF_3$ | H | - | H | H | H |
| H | $OCF_3$ | H | - | H | H | CN |
| H | $OCF_3$ | H | - | H | H | $CH=CH_2$ |

Tabelle 4 - Fortsetzung

| $R^1$ | $R^2$ | $R^3$ | n | $R^4$ | $R^5$ | $R^6$ |
|---|---|---|---|---|---|---|
| H | $OCHF_2$ | H | - | H | H | H |
| $OCH_3$ | $OCH_3$ | 3-$OCH_3$ | 1 | H | H | $CF=CF_2$ |
| F | H | H | - | H | H | $CH(CH_3)_2$ |
| H | $SCF_3$ | H | - | H | H | H |
| Cl | Cl | Cl(3,5) | 2 | H | H | H |
| H | $CH=Cl_2$ | H | - | H | H | H |
| H | H | $CH_2-CH=CH_2$ (3,5) | 2 | H | H | $CH=CH_2$ |
| Br | H | H | - | H | H | $CH(CH_3)_2$ |
| H | O-tert.-$C_4H_9$ | H | - | H | H | H |
| H | $OCH_3$ | 3-Br | 1 | H | H | CN |
| H | $OCF_2-CHF_2$ | H | - | H | H | H |
| H | O-$(CH_2)_5-CH_3$ | H | - | H | H | $CH_3$ |
| F | F | H | - | H | H | CN |
| H | F | 3-$CH_3$ | 1 | H | H | H |
| H | H | H | - | $CH(CH_3)_2$ | H | $CH(CH_3)_2$ |
| F | H | 6-F | 1 | H | H | H |
| H | $OCH_3$ | $OCH_3$(3,5) | 2 | H | H | H |
| F | H | H | - | H | H | C≡CH |
| F | H | H | - | H | H | CN |
| Cl | H | 6-F | 1 | H | H | CN |

Zur Herstellung der erfindungsgemäßen Wirkstoffe (Pyrethroide; d.h. Ester) ist das Folgende zu sagen:

Die vorstehend beschriebenen Benzylfurylcarbinole werden mit einer 3,3-Di-methylcyclopropan-1-carbonsäure, die in 2-Stellung durch eine 2',2'-disubstituierte Vinylgruppe substituiert ist, verestert. Diese Säuren und ihre Derivate sind beispielsweise in Wegler, Chemie der Pflanzenschutz- und Schädlingsbekämpfungsmittel, Band 7, Springer Verlag, Berlin, Heidelberg, New York 1981 beschrieben.

Beispielsweise seien folgende Säuren genannt:

A 1: 2-(2',2'-Dimethylvinyl)-3,3-dimethylcyclopropancarbonsäure-1
A 2: 2-(2',2'-Dichlorvinyl)-3,3-dimethylcyclopropancarbonsäure
A 8: 2-(2'-Trifluormethyl-2'-chlorvinyl)-3,3-dimethylcyclopropancarbonsäure
A 9: 2-(2',2'-Dibromvinyl)-3,3-dimethylcyclopropancarbonsäure
A 10: 2-(2'-Trifluormethyl-2'-fluorvinyl-3,3-dimethylcyclopropancarbonsäure
A 11: 2-(2'-Methyl-2'-methoxycarbonylvinyl)-3,3-dimethylcyclopropancarbonsäure
A 12: 2-(2',2'-Difluorvinyl)-3,3-dimethylcyclopropancarbonsäure
A 15: 2-[2'(4'-Chloryphenyl)-2'-chlorvinyl]-3,3-dimethylcyclopropancarbonsäure
A 16: 2-(2'-Vinylvinyl)-3,3-dimethylcyclopropancarbonsäure
A 17: 2-(2',2'-Bistrifluormethyl-vinyl)-3,3-dimethylcyclopropancarbonsaure.

Es versteht sich, daß die Verbindungen der Formel I in jedem Falle in Form von einheitlichen Diastereomeren, mindestens einem Paar optischer Antipoden, in vielen Fällen auch in Form von Diastereomeren-Vielfachen vorkommen und als Wirkstoffe angewandt werden können, die je nach den

Ausgangsstoffen und den Umsetzungsbedingungen in einheitlicher Form oder als Gemische auftreten. Die Gemische können in üblicher Weise in ihre sterisch einheitlichen Bestandteile aufgetrennt werden; ihre biologische Wirkung ist in Einzelfällen von ihrer sterischen Konfiguration abhängig.

Die Herstellung der erfindungsgemäßen Ester der Formel I ist durch entsprechende Abwandlung der folgenden Beispiele möglich:

Herstellbeispiel 1

2(2',2'-Dibromvinyl)-3,3-dimethylcylopropan-1-carbonsäure-2(2'-chlor-6'-fluorbenzyl)-furyl-4-methylester (A 9-Ester)
4,9 g 2-Chlor-6-fluor-benzylfurylcarbinol-4 und 2,5 g Triethylamin in 100 ml Toluol werden bei 0 - 15°C tropfenweise mit 6,45 g len 2-(2',2'-Dibromvinyl-3,3-dimethylcyclopropancarbonsäurechlorid versetzt. Nach Abklingen der exothermen Reaktion wird 5 Stunden bei 50°C nachgerührt. Nach dem Abkühlen wird filtriert und die Toluolphase mit Wasser gewaschen und über Natriumsulfat getrocknet. Das Lösungsmittel wird abgezogen und der Rückstand bei 60°C andestilliert. Man erhält 10,5 g eines hellgelben Öls, entsprechend einer Ausbeute von 99 %.

300-MHz-NMR-Spektrum (ppm, $CDCl_3$: 1,07 (3H); 1,12 (3H); 1,31 (1H); 2,08 (1H); 4,06 (2H); 4,97 (2H); 6,02 (1H); 6,08 (1H); 6,8 - 7,18 (3H + 1H).

Herstellbeispiel 2

2(2',2'-Dibromvinyl)-3,3-dimethylcyclopropan-1-carbonsäure-2-( 4'-fluorbenzyl)-furyl-4-cyanomethylester (A 9-Ester)
2 g 3-Formyl-5-(4'-fluorbenzyl)-furan in 50 ml Ether werden mit einer Lösung von 0,8 g Kaliumcyanid in 10 ml Wasser und 3,5 g 2,2-Dimethyl-4-(2',2'-dibromvinyl)-cyclopropancarbonsäurechlorid versetzt. Danach wird 15 Stunden bei Raumtemperatur gerührt, die organische Phase abgetrennt, mit Wasser gewaschen und nach Trocknung über Natriumsulfat vom Lösungsmittel befreit. Man erhält 4,9 g eines Öls, das nach säulenchromatographischer Reinigung 2,2 g reine Verbindung ergibt.

200-MHz-NMR-Spektrum in $CDCl_3$; ppm-Werte: 1,1 - 1,4 (6H); 1,8 (1H); 2,15 (1H); 3,95 (2H); 6,16 (1H); 6,3 (1H); 6,2 - 6,75 (1H); 6,95 -7,7 (5H).

Nach den beschriebenen Verfahren wurden die in der folgenden Tabelle genannten erfindungsgemäßen Ester hergestellt, die durch die Angabe physikalischer Eigenschaften näher charakterisiert sind; die übrigen Verbindungen der Tabelle können unter Verwendung entsprechender Ausgangsstoffe leicht erhalten werden:

| Nr. | A | $R^6$ | $R^4$ | $R^5$ | $R^1$ | $R^2$ | $R^3$ | n | NMR-Daten (MHz, 2M) ppm |
|---|---|---|---|---|---|---|---|---|---|
| 3 | A 2 | H | H | H | Cl | H | 2-F | 1 | (300, $CDCl_3$) 1,05 - 1,4 (6H); 1,6 - 1,9 (1H); 1,95 - 2,4 (1H); 4,15 (2H); 4,9 (2H); 5,6 - 6,25 (1H + 1H); 7,0 (1H); 7,1 - 7,4 (3H) |
| 4 | A 9 | H | H | H | Cl | H | H | 0 | (300, $CDCl_3$) 1,18 (3H); 1,25 (3H); 1,65 (1H); 2,18 (1H); 4,06 (2H); 4,95 (2H); 6,05 (1H); 6,17 (1H); 7,06 - 7,4 (4H + 1H) |
| 5 | A 8 | H | H | H | H | F | H | 0 | |
| 6 | A 8 | H | H | H | H | F | 3-F | 1 | |
| 7 | A 2 | H | H | H | Cl | H | H | 0 | (300, $CDCl_3$) 1,1 - 1,4 (6H); 1,6 - 1,81 (1H); 1,95 - 2,3 (1H); 1,05 (2H); 4,9 (2H); 5,6 - 6,25 (1H); 6,05 (1H); 7,1 - 7,4 (5H) |

| Nr. | A | $R^6$ | $R^4$ | $R^5$ | $R^1$ | $R^2$ | $R^3$ | n | NMR-Daten (MHz, 2M) ppm |
|---|---|---|---|---|---|---|---|---|---|
| 8 | A 1 | H | H | H | Cl | H | H | 0 | (300, ($CDCl_3$) 1,1 - 1,3 (6H); 1,4 (1H); 1,7 (6H); 1,8 - 2,1 (1H); 4,05 (2H); 5,86 (2H); 4,8 - 5,4 (1H); 6,05 (1H); 7,11 - 7,4 (6H) |
| 9 | A 1 | H | H | H | H | H | 3-$CF_3$ | 1 | (300, $CDCl_3$) 1,1 - 1,4 (6H 1,7 (6H); 1,8 - 2,2 (1H); 4,0 (2H); 4,86 (2H); 4,95 - 5,4 (1H); 6,08 (1H); 7,2 - 7,6 (5H) |
| 10 | A 2 | H | $CH_3$ | $CH_3$ | H | H | H | 0 | (200, $CDCl_3$) 1,15 - 1,3 (6H); 1,6 - 1,7 (6H); 1,7 - 2,4 (1H + 1H); 4,95 (2H); 5,6 - 6,35 (1H + 1H); 7,15 - 7,4 (6H) |
| 11 | A 2 | H | H | H | H | F | H | 0 | (360, $CDCl_3$) 1,1 - 1,4 (6H); 1,5 - 2,2 (2H); 3,9 (2H); 4,9 (2H); 5,6 - 6,3 (1H); 6,05 (1H); 6,9 - 7,3 (5H) |
| 12 | A 2 | H | H | H | H | H | 3-$CF_3$ | 1 | (300, $CDCl_3$) 1,08 - 1,2 (6H); 1,58 - 1,86 (1H); 1,98 - 2,3 (1H); 4,0 (2H); 4,93 (2H); 5,6 6,15 (1H); 6,04 (1H); 7,25 - 7,6 (5H) |
| 13 | A 10 | H | H | H | $CF_3$ | H | H | 0 | |
| 14 | A 8 | H | H | H | H | $CF_3$ | H | 0 | |

| Nr. | A | $R^6$ | $R^4$ | $R^5$ | $R^1$ | $R^2$ | $R^3$ | n | NMR-Daten (MHz, 2M) ppm |
|---|---|---|---|---|---|---|---|---|---|
| 15 | A 1 | H | H | H | H | H | 3-F | 1 | (300, $CDCl_3$) 1,05 - 1,33 (6H); 1,6 - 1,8 (6H); 1,86 (1H); 2,07 (1H); 3,94 (2H); 4,9 (2H); 4,9 - 5,4 (1H); 6,05 (1H); 6,8 - 7,4 (5H) |
| 16 | A 9 | H | H | H | H | Cl | H | 0 | (200, $CDCl_3$) 1,15 - 1,3 (6H); 2,15 (1H); 2,4 (1H); 3,9 (2H); 4,9 (2H); 6,05 (1H); 6,1 - 6,5 (1H); 7,1 - 7,4 (5H) |
| 17 | A 2 | H | H | H | H | Cl | H | 0 | (200, $CDCl_3$) 1,1 - 1,4 (6H); 1,78 (1H); 2,2 (1H); 3,9 (2H); 4,92 (2H); 5,6 - 6,2 (1H); 6,05 (1H); 7,1 - 7,4 (5H) |
| 18 | A 2 | H | H | H | F | H | H | 0 | (360, $CDCl_3$) 1,05-1,35 (6H); 1,7 (1H); 2,1 (1H); 3,95 (2H); 4,9 (2H); 5,5-6,3 (1H); 6,1 (1H); 6,9-7,4 (5H) |
| 19 | A 1 | H | $CH_3$ | $CH_3$ | H | H | H | 0 | (300, $CDCl_3$) 1,15 (3H); 1,22 (3H); 1,55 - 1,8 (13H); 1,99 (1H); 4,9 (2H); 6,18 (1H); 7,1 - 7,4 (6H) |
| 20 | A 9 | H | $CH_3$ | $CH_3$ | H | H | H | 0 | (300, $CDCl_3$) 1,2 (3H); 1,25 (3H); 1,57 - 1,75 (7H); 2,1 (1H); 4,95 (2H); 6,17 (1H); 6,18 - 6,8 (1H); 7,1 - 7,4 (6H) |

| Nr. | A | $R^6$ | $R^4$ | $R^5$ | $R^1$ | $R^2$ | $R^3$ | n | NMR-Daten (MHz, 2M) ppm |
|---|---|---|---|---|---|---|---|---|---|
| 21 | A 1 | H | H | H | H | F | H | 0 | (360, $CDCl_3$) 1,0 - 1,3 (6H); 1,7 - 1,9 (6H); 1,95 (1H); 2,35 (1H); 3,9 (2H); 4,9 (2H);4,9 - 5,4 (1H); 6,05 (1H); 6,9 - 7,4 (5H) |
| 22 | A 1 | H | H | H | F | H | H | 0 | (360,$CDCl_3$) 1,0 - 1,25 (6H); 1,6 - 1,8 (6H); 1,85 (1H); 2,0 (1H); 3,9 (2H); 4,85 (2H); 4,9 - 5,3 (1H); 6,05 (1H); 6,9 - 7,4 (5H) |
| 23 | A 2 | H | H | H | H | H | 3-F | 1 | (360,$CDCl_3$)1,0 - 1,4 (6H); 1,74 (1H); 2,12 (1H); 3,9 (2H); 4,9 (2H); 5,6 - 6,3 (1H); 6,1 (1H); 6,1 (1H); 6,8 - 7,3 (5H) |
| 24 | A 9 | H | H | H | H | F | H | 0 | (200,$CDCl_3$)1,1 - 1,4 (6H); 1,77 (1H);2,1 (1H); 3,96 (2H); 4,95 (1H); 6,05 (1H); 6,1 - 6,8 (1H); 6,9 - 7,45 (5H); |
| 25 | A 2 | H | H | H | H | F | 3-F | 1 | |
| 26 | A 2 | H | H | H | F | F | 3-F | 1 | |
| 27 | A 9 | H | H | H | F | F | F | 3 | |
| | A 9 | H | H | H | F | F | F | 2 | |

| Nr. | A | $R^6$ | $R^4$ | $R^5$ | $R^1$ | $R^2$ | $R^3$ | n | NMR-Daten (MHz, 2M) ppm |
|---|---|---|---|---|---|---|---|---|---|
| 29 | A 8 | H | H | H | H | F | 3-F | 1 | |
| 30 | A 9 | Ethinyl | H | H | H | F | H | 0 | (300,$CDCl_3$) 1,1 - 1,4 (6H); 1,8 (1H); 2,08 (1H); 2,58 (1H); 3,92 (2H); 6,1 (1H); 6,37 (1H); 6,15 - 6,9 (1H); 6,95 - 7,5 (5H) |
| 31 | A 9 | CN | H | H | H | F | H | 0 | (200,$CDCl_3$) 1,1 - 1,4 (6H); 1,8 (1H); 2,1 (1H); 3,95 (2H); 6,16 (1H); 6,3 (1H); 6,2 - 6,75 (1H); 6,95 - 7,7 (5H) |
| 32 | A 9 | H | H | H | Cl | Cl | H | 0 | (270, $CDCl_3$) 1,1 - 1,35 (6H); 1,62 (1H); 2,06 (1H); 4,0 (2H); 4,92 (2H); 6,05 (1H); 6,1 - 6,7 (1H); 7,1 - 7,4 (4H) |
| 33 | A 1 | H | H | H | Cl | Cl | H | 0 | (270,$CDCl_3$) 1,08 - 1,3 (6H); 1,5 (1H); 1,6 - 1,75 (6H); 1,97 (1H); 4,0 (2H); 4,86 (2H); 4,9 - 5,4 (1H); 6,05 (1H); 7,05 - 2,7 (4H) |

| Nr. | A | $R^6$ | $R^4$ | $R^5$ | $R^1$ | $R^2$ | $R^3$ | n | NMR-Daten (MHz, 2M) ppm |
|---|---|---|---|---|---|---|---|---|---|
| 34 | A 9 | $CH_2$=CH- | H | H | H | F | H | 0 | (300, $CDCl_3$ 1,1 - 1,38 (3H); 1,78 (1H); 2,05 (1H); 4,9 (2H); 4,1 - 4,2 (2H); 5,9 - 6,23 (4H); 6,91 - 7,37 (5H) |
| 35 | A 9 | H | H | H | H | H | 3-Cl | 1 | (300,$CDCl_3$) 1,1 - 1,4 (6H); 1,78 (1H); 2,09 (1H); 3,91 (2H); 4,92 (2H); 6,07 (1H); 6,1 - 6,8 (1H); 7,08 - 7,4 (5H) |
| 36 | A 2 | H | H | H | Cl | Cl | H | 0 | (270,$CDCl_3$) 1,05 - 1,35 (6H); 1,71 (1H); 2,1 (1H); 4,0 (2H); 4,92 (2H); 6,08 (1H); 5,04 - 6,3 (1H); 7,05 - 7,45 (4H) |
| 37 | A 1 | H | H | H | H | H | 3-Cl | 1 | (300, $CDCl_3$) 1,1 - 1,3 (6H); 1,6 (1H); 1,55 - 1,8 (6H); 1,95 (1H); 3,88 (2H);4,92 (2H); 4,9 - 5,4 (1H); 6,05 (1H); 7,04 - 7,4 (5H) |
| 38 | A 2 | $CH_2$=CH- | H | H | H | F | H | 0 | (300, $CDCl_3$) 1,1 - 1,38 (6H); 1,77 (1H); 2,16 (1H); 3,98 (2H); 5,2 - 5,65 (2H); 5,86 - 6,16 (4H); 6,9 - 7,35 (5H) |

| Nr. | A | $R^6$ | $R^4$ | $R^5$ | $R^1$ | $R^2$ | $R^3$ | n | NMR-Daten (MHz, 2M) ppm |
|---|---|---|---|---|---|---|---|---|---|
| 39 | A 2 | H | H | H | H | H | 3-Cl | 1 | (300, $CDCl_3$) 1,1 - 1,3 (6H); 1,74 (1H); 2,17 (1H); 3,9 (2H); 4,9 (2H); 6,05 (1H); 5,55 - 6,3 (1H); 7,05 - 7,4(5H) |
| 40 | A 9 | H | H | $CH_3$ | H | H | H | 0 | (200, $CDCl_3$) 1,2 (3H); 1,3 (3H); 1,67 (1H); 2,2 (1H); 4,1 (1H); 4,92 (2H); 6,1 (1H); 6,1 (1H); 6,18 (1H); 7,2 - 7,4 (6H) |
| 41 | A 2 | H | H | $CH_3$ | [illegible] | H | H | 0 | (200, $CDCl_3$) 1,1 - 1,4 (6H); 1,1 (3H), 1,92 (1H); 2,25 (1H); 4,1 (1H); 4,95 (2H); 6,1 (1H); 5,5 - 6,3 (1H); 7,2 - 7,4 (6H) |
| 42 | A 2 | HC≡C- | H | H | H | F | H | 0 | (300, $CDCl_3$) 1,06 - 1,37 (6H); 1,76 (1H), 2,17 (1H); 2,75 (1H); 3,9 (2H); 5,55 - 6,4 (3H); 6,95 - 7,52 (5H). |

Anwendungsbeispiele

Kontaktwirkung auf Stubenfliegen (Musca domestica) Applikationstest

4-Tage alte Imagines erhalten in leichter $CO_2$-Narkose 1 µl der acetonischen Wirkstofflösung auf das ventrale Abdomen appliziert. Hierzu wird eine Mikrometerspritze verwendet.

Je 20 Versuchstiere mit gleicher Behandlung bringt man in einen Cellophanbeutel (ca. 500 ml).

Nach 4 Stunden zählt man die Tiere in Rückenlage aus und ermittelt graphisch die LD 50.

Die Verbindungen der Beispiele 11, 17, 18 und 23 erzielen bei diesem Versuch eine $LD_{50}$ zwischen 0,005 µm/Fliege und 0,008 µm/Fliege.

Die Vergleichsmittel I, II und III erzielen 0,01, 0,032 bzw. 0,016 µg/Fliege.

Kontaktwirkung auf Moskito-Larven (Aedes aegypti)

200 ml Leitungswasser werden mit der Wirkstoffaufbereitung versetzt und darauf mit 30-40 Moskito-Larven im 4. Larvenstadium besetzt.

Die Versuchstemperatur beträgt 20 ° C. Nach 24 Stunden wird die Wirkung ermittelt.

Hierbei erzielen die Wirkstoffe des Beispiels 11, 17, 21, 23, 24, 35 und 39 100 % Mortalität bei Anwendungskonzentrationen zwischen 0,002 und 0,02 ppm, während Vergleichsmittel I, II und III erst in einer Konzentration von 0,01 bis 0,02 ppm vergleichbar sind.

Plutella maculipennis; Kohlschaben-Raupen; Fraß- und Kontaktwirkung

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung. Dabei erzielen die Verbindungen der Beispiele 3, 4, 7, 11, 12, 15, 17, 21, 22, 23, 39, 40 und 41 eine mindestens zweifache Wirkung, gemessen am Vergleichsmittel I.

## Patentansprüche

**1.** 2-Benzylfurylverbindungen der allgemeinen Formel I

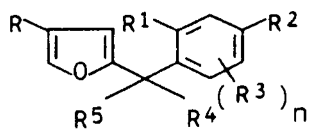

(I),

in der

| | |
|---|---|
| $R^1$, $R^2$ und $R^3$ | gleiche oder verschiedene Substituenten der Bedeutung Wasserstoff, Halogen, Alkyl, Halogenalkyl, Alkoxy, Halogenalkoxy, Halogenalkylthio, Alkenyl oder Halogenalkenyl mit jeweils bis zu 6 C-Atomen sind, wobei n die Zahl 1, 2 oder 3 ist, falls $R^3$ vom Wasserstoff verschieden ist, |
| $R^4$ und $R^5$ | Wasserstoff oder Alkyl mit bis zu 6 C-Atomen, |
| R | die Bedeutung -CHO oder $CHR^6OA$ hat, wobei |
| $R^6$ | für Wasserstoff, Cyano, Alkyl, Alkenyl, Halogenalkenyl oder Alkinyl mit jeweils bis zu 6 Kohlenstoffatomen oder die Carbonamidgruppe steht und |
| A | die Acylgruppe einer 3,3-Dimethylcyclopropan-1-carbonsäure bedeutet, die in 2-Stellung durch eine 2',2'-disubstituierte Vinylgruppe substituiert ist, |

mit der Maßgabe, daß, wenn A den Rest der 3,3-Dimethylcyclopropan-1-carbonsäure bedeutet, die in 2-Stellung eine 2',2'-Dimethylvinylgruppe trägt, $R^2$ nicht Methyl, Chlor, Brom oder Methoxy und $R^1$ und $R^3$ nicht Wasserstoff sind und ferner $R^1$ nicht Wasserstoff oder Methyl bedeutet, wenn $R^2$ und $R^3$ gleichzeitig für Wasserstoff stehen.

**2.** 2-Benzylfurylverbindungen der Formel I gemäß Anspruch 1, wobei A den Acylrest einer der folgenden Säure bedeutet:
2-(2',2'-Dimethylvinyl)-3,3-dimethylcyclopropancarbonsäure,
2-(2',2'-Dichlorvinyl)-3,3-dimethylcyclopropancarbonsäure,
2-(2'-Trifluormethyl-2'-chlorvinyl)-3,3-dimethylcyclopropancarbonsäure,
2-(2',2'-Dibromvinyl)-3,3-dimethylcyclopropancarbonsäure,
2-(2'-Trifluormethyl-2'-fluorvinyl)-3,3-dimethylcyclopropancarbonsäure,
2-(2'-Methyl-2'-methoxycarbonylvinyl)-3,3-dimethylcyclopropancarbonsäure,
2-(2',2'-Difluorvinyl)-3,3-dimethylcycopropancarbonsäure.

**3.** Verfahren zur Herstellung von Isoxazolinen der allgemeinen Formel IV,

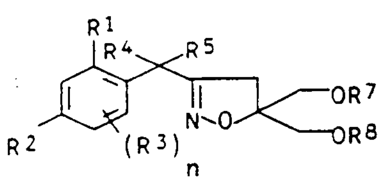

in der

| | |
|---|---|
| $R^1$ bis $R^5$ | und n die in Anspruch 1 angegebene Bedeutung haben, und |
| $R^7$ und $R^8$ | gleich oder verschieden sind und für Wasserstoff, Alkyl, Alkylcarbonyl, Arylcarbonyl oder eine andere abspaltbare Schutzgruppe stehen, mit der Maßgabe, daß $R^7$ und $R^8$ |

nicht für Acetyl stehen, wenn
$R^1$, $R^2$ und $R^3$ Wasserstoff;
$R^1$ und $R^2$ Halogen und $R^3$ Wasserstoff;
$R^1$ und $R^2$ Wasserstoff, n = 1 und $R^3$ Trifluormethyl bedeuten;
sowie ferner mit der Maßgabe, daß $R^7$ und $R^8$ nicht für Wasserstoff stehen, wenn $R^1$, $R^2$ und $R^3$ Wasserstoff oder $R^1$ und $R^3$ Wasserstoff und $R^2$ Halogen bedeuten;
dadurch gekennzeichnet, daß man einen entsprechenden Phenylacetaldehyd der allgemeinen Formel V

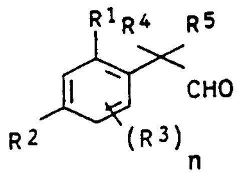

(V),

in das entsprechende Oxim überführt, dieses zum Nitriloxid umsetzt und mit einer entsprechenden olefinischen Verbindung VI

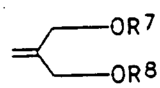

zur Reaktion bringt.

4. Verfahren zur Herstellung von Estern der allgemeinen Formel I gemäß Anspruch 1, dadurch gekennzeichnet, daß man ein entsprechendes Carbinol Ia

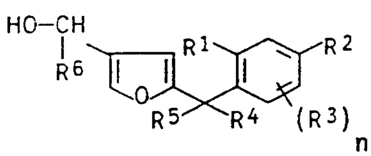

(Ia),

in an sich bekannter Weise mit einer der in Anspruch 1 genannten Carbonsäuren AOH bzw. deren reaktionsfähigen Derivaten umsetzt.

5. Verwendung der Ester der allgemeinen Formel I gemäß Anspruch 1 zur Herstellung von Insektiziden durch Zusatz von Formulierungshilfmitteln oder von anderen Wirkstoffen.

6. Verfahren zur Bekämpfung von Insekten oder anderen tierischen Schädlingen, dadurch gekennzeichnet, daß man einen Wirkstoff der allgemeinen Formel I oder ein Insektizid, das gemäß Anspruch 5 erhalten worden ist, auf die Schädlinge oder deren Lebensraum einwirken läßt.

## Claims

1. A 2-benzylfuryl compound of the formula I

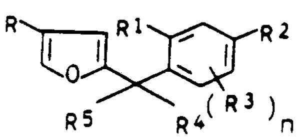

(I)

where $R^1$, $R^2$ and $R^3$ are identical or different substituents and are each hydrogen, halogen, alkyl, haloalkyl, alkoxy, haloalkoxy, haloalkylthio, alkenyl or haloalkenyl, each of not more than 6 carbon atoms, n being 1, 2 or 3 where $R^3$ is not hydrogen, and $R^4$ and $R^5$ are each hydrogen or alkyl of not more than 6 carbon atoms, R is -CHO or $CHR^6OA$, $R^6$ is hydrogen, cyano, alkyl, alkenyl, haloalkenyl or alkynyl, each of not more than 6 carbon atoms, or carboxamide, and A is the acyl group of 3,3-dimethylcyclopropane-1-carboxylic acid which is substituted in the 2-position by a 2',2'-disubstituted vinyl group, with the proviso that $R^2$ is not methyl, chlorine, bromine or methoxy and $R^1$ and $R^3$ are not hydrogen when A is the radical of 3,3-dimethylcyclopropane-1-carboxylic acid which carries a 2',2'-dimethylvinyl group in the 2-position, and furthermore $R^1$ is not hydrogen or methyl when $R^2$ and $R^3$ are simultaneously hydrogen.

**2.** A 2-benzylfuryl compound of the formula I as claimed in Claim 1, in which A is the acyl radical of one of the following acids: 2-(2',2'-dimethylvinyl)-3,3-dimethylcyclopropanecarboxylic acid, 2-(2'2'-dichlorovinyl)-3,3-dimethycyclopropanecarboxylic acid, 2-(2'-trifluoromethyl-2'-chlorovinyl)-3,3-dimethylcyclopropanecarboxylic acid, 2-(2',2'-dibromovinyl)-3,3-dimethylcyclopropanecarboxylic acid, 2-(2'-trifluoromethyl-2'-fluorovinyl)-3,3-dimethylcyclopropanecarboxylic acid, 2-(2'-methyl-2'methoxycarbonylvinyl)-3,3-dimethylcyclopropanecarboxylic acid and 2-(2',2'-difluorovinyl)-3,3-dimethylcyclopropanecarboxylic acid.

**3.** A process for the preparation of an isoxazoline of the formula IV

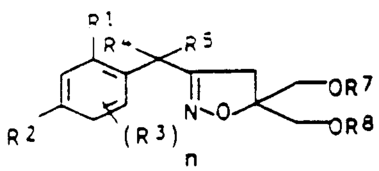

(IV)

where $R^1$ to $R^5$ and n have the meanings stated in Claim 1 and $R^7$ and $R^8$ are identical or different and are each hydrogen, alkyl, alkylcarbonyl, arylcarbonyl or another protective group which can be eliminated, with the proviso that $R^7$ and $R^8$ are not acetyl when $R^1$, $R^2$ and $R^3$ are hydrogen; $R^1$ and $R^2$ are halogen and $R^3$ is hydrogen; $R^1$ and $R^2$ are hydrogen, n is 1 and $R^3$ is trifluoromethyl; and furthermore with the proviso that $R^7$ and $R^8$ are not hydrogen when $R^1$, $R^2$ and $R^3$ are hydrogen or $R^1$ and $R^3$ are hydrogen and $R^2$ is halogen, wherein an appropriate phenylacetaldehyde of the formula V

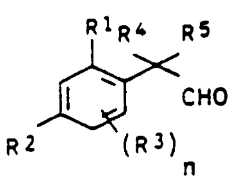

(V)

is converted to the corresponding oxime, the latter is converted to the nitrile oxide, and the product is reacted with an appropriate olefinic compound VI

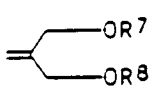

(VI).

**4.** A process for the preparation of an ester of the formula I as claimed in Claim 1, wherein an appropriate carbinol Ia

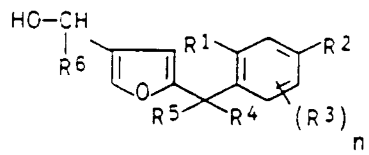

(Ia)

is reacted, in a conventional manner, with a carboxylic acid AOH as mentioned in Claim 1, or one of its reactive derivatives.

5. Use of an ester of the formula I as claimed in Claim 1, for the preparation of an insecticide by adding formulation assistants or other active ingredients.

6. A method of controlling insects or other animal pests, wherein an active ingredient of the formula I or an insecticide obtained according to Claim 5 is allowed to act on the pests or their habitat.

**Revendications**

1. Dérivés 2-benzylfuryliques de formule générale I

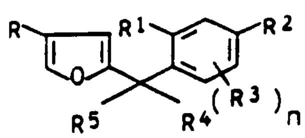

(I),

dans laquelle
$R^1$, $R^2$ et $R^3$, ayant des significations identiques ou différentes, représentent chacun l'hydrogène, un halogène, un groupe alkyle, halogénoalkyle, alcoxy, halogénoalcoxy, halogénoalkylthio, alcényle ou halogénoalcényle, contenant chacun jusqu'à 6 atomes de carbone, n étant égal à 1, 2 ou 3 lorsque $R^3$ a une signification autre que l'hydrogène,
$R^4$ et $R^5$ représentent l'hydrogène ou des groupes alkyle contenant jusqu'à 6 atomes de carbone,
R représente -CHO ou $CHR^6OA$ dans lequel
$R^6$ représentant l'hydrogène, un groupe cyano, alkyle, alcényle, halogénoalcényle, ou alcynyle contenant chacun jusqu'à 6 atomes de carbone, ou le groupe carboxamide, et
A représentant le radical acyle d'un acide 3,3-diméthylcyclopropane-1-carboxylique substitué en position 2 par un groupe vinyle lui-même 2',2'-disubstitué, sous réserve que, lorsque A représente le radical de l'acide 3,3-diméthylcyclopropane-1-carboxylique portant en position 2 un groupe 2',2'-diméthylvinyle, $R^2$ ne peut représenter un groupe méthyle, le chlore, le brome ou un groupe méthoxy et $R^1$ et $R^3$ ne peuvent représenter l'hydrogène, et en outre que $R^1$ ne peut représenter l'hydrogène ou un groupe méthyle lorsque $R^2$ et $R^3$ représentent tous deux l'hydrogène.

2. Dérivés 2-benzylfuryliques de formule I de la revendication 1, dans lesquels A représente le radical acyle de l'un des acides suivants :
acide 2-(2',2'-diméthylvinyl)-3,3-diméthylcyclopropane-carboxylique,
acide 2-(2',2-dichlorovinyl)-3,3-diméthylcyclopropane-carboxylique,
acide 2-(2'trifluorométhyl-2'-chlorovinyl)-3,3-diméthylcyclopropane-carboxylique,
acide 2-(2',2'-dibromovinyl)-3,3-diméthylcyclopropane-carboxylique,
acide 2-(2'-trifluorométhyl-2'-fluorovinyl)-3,3-diméthylcyclopropane-carboxylique,
acide 2-(2'-méthyl-2'-méthoxycarbonylvinyl)-3,3-diméthylcyclopropane-carboxylique,
acide 2-(2',2'-difluorovinyl)-3,3-diméthylcyclopropane-carboxylique.

3. Procédé de préparation des isoxazolines de formule générale IV

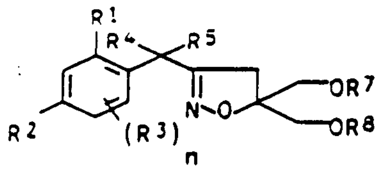

dans laquelle
$R^1$ à $R^5$ et n ont les significations indiquées dans la revendication 1, et
$R^7$ et $R^8$, ayant des significations identiques ou différentes, représentent chacun l'hydrogène, un groupe alkyle, alkylcarbonyle, arylcarbonyle ou un autre groupe protecteur scindable, sous réserve que $R^7$ et $R^8$ ne peuvent représenter des groupes acétyle lorsque
$R^1$, $R^2$ et $R^3$ représentent l'hydrogène ;
$R^1$ et $R^2$ représentent des halogènes et $R^3$ l'hydrogène ;
$R^1$ et $R^2$ représentent l'hydrogène, n est égal à 1 et $R^3$ représentent l'hydrogène et $R^2$ un halogène ;
caractérisé en ce que l'on convertit un phénylacétaldéhyde correspondant de formule générale V

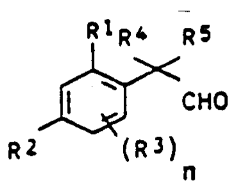

(V),

en l'oxime correspondante, qu'on convertit en oxyde de nitrile qu'on fait réagir avec un dérivé oléfinique correspondant VI

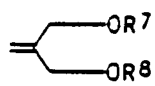

4. Procédé de préparation d'esters de formule générale I de la revendication 1, caractérisé en ce que l'on fait réagir un carbinol correspondant la

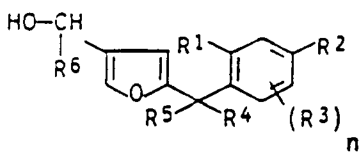

(Ia),

de manière connue en soi avec l'un des acides carboxyliques AOH mentionnés dans la revendication 1 ou ses dérivés réactifs.

5. Utilisation des esters de formule générale I de la revendication 1, pour la préparation de produits insecticides par adjonction de produits auxiliaires de formulation ou d'autres substances actives.

6. Procédé pour combattre les insectes ou d'autres parasites animaux, caractérisé en ce que l'on fait agir sur les parasites ou leur habitat une substance active de formule générale I ou un produit insecticide obtenu selon la revendication 5.